# EUROPEAN PATENT APPLICATION

(11) **EP 4 732 836 A1**
(43) Date of publication of application: **29.04.2026**
(21) Application number: 24383164.1
(22) Date of filing: 22.10.2024
(51) Int. Cl.: A61K 31/365, A61K 31/593, A61P 1/16

(54) **AG5 COMPOUND FOR USE IN THE TREATMENT OF IN FATTY LIVER DISEASES**

(71) Applicant: Consejo Superior De Investigaciones Científicas, 28006 Madrid (ES); Universitat Politècnica de València, 46022 Valencia (ES)
(72) Inventor: Botella Asunción, Pablo, 46022 Valencia (ES); Benlloch Baviera, José María, 46022 Valencia (ES); Montoya Gonzalez, María, 28040 Madrid (ES); Quesada Gómez, José Manuel, 28006 Madrid (ES)
(74) Representative: Pons IP

(57) **Abstract**

The present invention refers to AG5 compound, and compositions or combined preparations comprising said compound, for use in the treatment and/or prevention of a fatty liver disease or disorder.

## Description

The invention belongs to the field of biomedicine and pharmaceuticals. In particular, the present invention relates to AG5 compound, and compositions or combined preparations comprising said compound, for use in the treatment and/or prevention of fatty liver diseases or disorders.

### BACKGROUND ART

Fatty liver diseases are characterized by the build-up and accumulation of excessive lipids in liver cells (steatosis), including disorders such as alcohol-induced fatty liver disease or nonalcoholic fatty liver disease (NAFLD).

These diseases are the most common hepatic disorders worldwide, and its prevalence continues to increase with the growing obesity epidemic (Vernon G, Baranova A, Younossi ZM. Systematic review: the epidemiology and natural history of non-alcoholic fatty liver disease and non-alcoholic steatohepatitis in adults. Aliment Pharmacol Ther. 2011 Aug;34(3):274-85). They are often asymptomatic and is usually discovered during routine laboratory tests with altered transaminase levels.

Effective treatments for fatty liver diseases remain insufficient, and to date, no effective therapeutic drug treatment is established for such patients.

Currently, fatty liver diseases such as NAFLD or non-alcoholic steatohepatitis (NASH), are primarily managed in early stages through lifestyle modification (e.g., physical exercise, weight loss, and healthy diet) which may encounter poor adherence. Losing weight addresses the conditions that contribute to nonalcoholic fatty liver disease. Bariatric surgery is also an option for those who need to lose a great deal of weight. Anti-diabetic medication, vitamins or dietary supplements can be useful for controlling the condition. For those who have cirrhosis due to NASH, liver transplantation may be an option.

On the other hand, alcoholic liver disease accounts for the majority of chronic liver diseases in Western countries. It encompasses a spectrum of liver manifestations of alcohol overconsumption, including fatty liver. Restricting alcohol intake is the primary treatment for this disease, and other treatment options include supportive care (e.g., healthy diet, vitamin supplements), use of corticosteroids have been proposed for use in severe cirrhosis, and sometimes liver transplantation.

Some documents disclosing compounds for the treatment of fatty liver diseases are: EP3206692B1, which discloses treatment of fatty liver disease using a class of pyrimidinedione cyclohexyl compounds; or EP4166137B1, disclosing the use of a compound (represented by a specific formula (1) in the description of said document) in the treatment of a fatty liver disease.

Therefore, there is a need for developing alternative therapeutics for the treatment and/or prevention of fatty liver diseases such as NAFLD, NASH and/or alcoholic liver disease.

### DESCRIPTION OF THE INVENTION

The inventors of the present invention have demonstrated that AG5 compound (also referred in the present document as AG-5), which is an andrographolide derivative known in the state of the art, alone or in combination with calcifediol (also referred in the present document as 25OH-D3), has the capacity to reduce the formation and/or accumulation of intracellular fatty vesicles, as well as the capacity to inhibit the expression of some genes involved in fat metabolism.

Particularly, as it is shown with further detail in the Examples, it was assessed the effect of the compound AG5, with or without calcifediol, on fat vesicle formation and adipogenic gene regulation, in two types of cell lines, being of particular relevance the effects shown in a steatosis model of HEPG2, a human hepatocytes cell line.

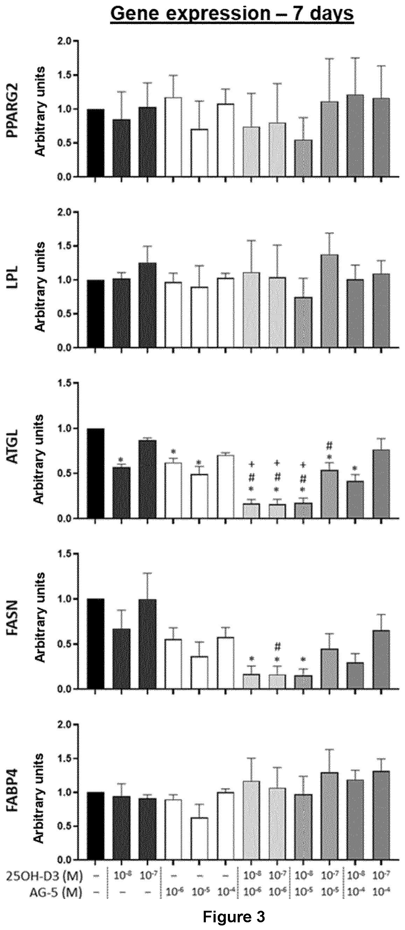

In Example 1, section 2.1., it is shown the fat accumulation reduction by AG5 and/or calcifediol treatment in bone marrow-derived mesenchymal stromal cells (MSCs) differentiated to adipocytes. Regarding the effect of AG5 and/or calcifediol in the gene expression of adipogenic differentiation marker genes, at 7 days of differentiation, a decrease in mRNA levels of ATGL, for which the most significant inhibition was shown, and a decrease in FASN were observed compared to untreated cells (Figure 3). The combination of calcifediol and AG5 produced a more significant inhibition of adipogenic gene expression than individual treatments.

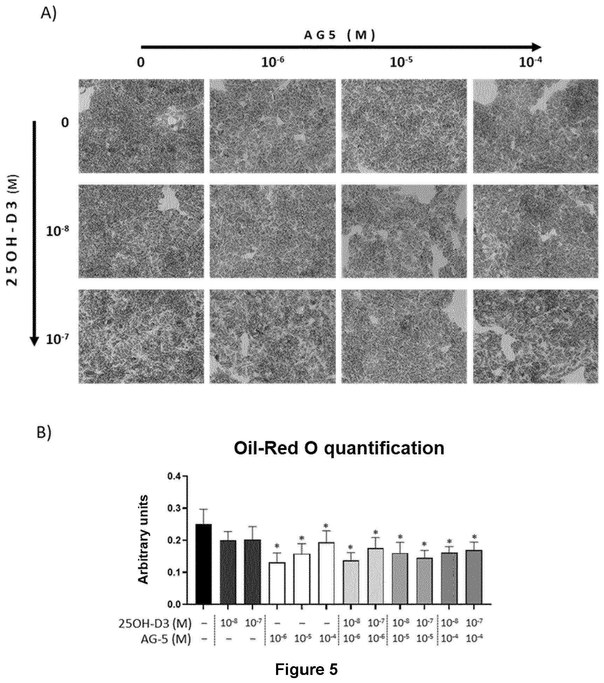

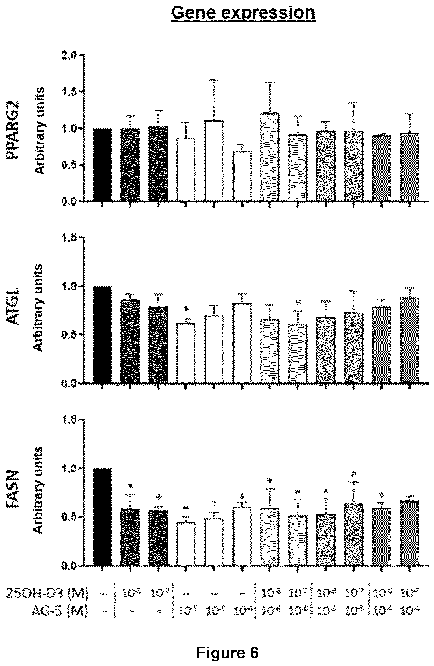

In Example 1, section 2.2., the effects of AG5, and/or calcifediol, in a steatosis model of HEPG2, a human hepatocytes cell line, were evaluated. The results show:
i) Treatment of HEPG2 with different combinations of AG5 and calcifediol, both during the induction of fatty acid fatty vesicle formation and after induction, decrease the accumulation of fatty vesicles: AG5, alone or in combination with calcifediol, produced a significant decrease in the accumulation of fat vesicles during the induction of fatty vesicle formation in HEPG2 cells, with significant differences when AG5 was present in all the evaluated treatments (Figure 5).
ii) The inhibition of the expression of genes involved in fat metabolism, mainly the one coding for fatty acid synthase (FASN), in HEPG2 cells treated with different combinations of AG5 and/or calcifediol, indicates that these compounds interfere in metabolic processes directed to the accumulation of fatty vesicles (Figure 6).

Based on these effects, the present invention concerns the therapeutic use of AG5, alone or in combination with calcifediol, in fatty liver disease, which is a prevalent liver condition characterised by the build-up of excessive fat (lipids) and its abnormal intracellular accumulation in hepatic cells.

As previously mentioned, AG5 is a known compound in the state of the art which has been described for the use in the treatment of pulmonary fibrosis associated with COVID-19 (WO2022090597A1) and pathologies involving inflammation associated with cytokine storm (WO2022090605A1), as well as it has been described its anti-inflammatory effect preserving adequately the innate immune system (Botella-Asunción P, Rivero-Buceta EM, et al. AG5 is a potent non-steroidal anti-inflammatory and immune regulator that preserves innate immunity. Biomed Pharmacother. 2023 Dec 31:169:115882. doi: 10.1016/j.biopha.2023.115882). Nevertheless, although these AG5 effects and therapeutic applications have been previously disclosed, its therapeutic use in fatty liver diseases, such as NAFLD or NASH, has not been previously documented. Hence, the present invention provides alternative therapeutics for the treatment and/or prevention of fatty liver diseases.

Thus, in a first aspect, the present invention refers to AG5 compound (14-deoxy-12(R,S)-sulfoandrographolide): 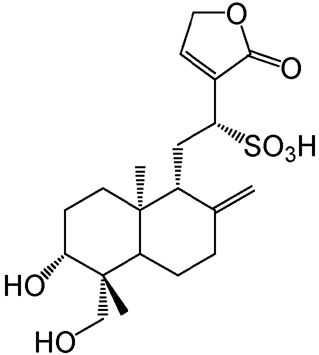 for use in the treatment and/or prevention of a fatty liver disease or disorder, or a condition caused by fatty liver in a subject, preferably a subject in need thereof.

The present invention also relates to the salts, preferably a pharmaceutically acceptable salt, hydrates and solvates of compound AG5.

A "pharmaceutically acceptable salt" refers to a salt of the compound that does not cause undue toxicity, irritation, allergic or similar response to the organism to which the compound is administered and does not impair the biological activity and properties of the compound.

"Hydrate" means a compound of the present invention containing a stoichiometric or non-stoichiometric amount of water bound by non-covalent intermolecular forces.

"Solvate" means a compound of the present invention or a salt thereof, comprising a stoichiometric or non-stoichiometric amount of a solvent bound by non-covalent intermolecular forces.

Furthermore, compound AG5 has chiral centers, which can give rise to various stereoisomers. The present invention relates to each of the individual stereoisomers, as well as to mixtures thereof.

### Fatty liver disease and related condition/disorders

As previously explained, and shown with more detail in Example section, AG5 (as well as AG5 in combination with calcifediol) decreases the formation and/or accumulation of fatty vesicles and inhibits the expression of some genes involved in fat metabolism, allowing AG5 (as well as AG5 in combination with calcifediol, as described in further detail later in this document) therapeutic application in fatty liver diseases/disorders and/or related conditions caused by fatty liver.

Fatty liver disease (FLD) is a prevalent liver condition that occurs when lipids accumulate in liver cells. The lipid accumulation causes cellular injury and makes the liver susceptible to further injuries. Fatty liver disease is characterized by the build-up of excessive fat (lipids) in liver cells, generally caused by abnormal retention of lipids by the liver cells (i.e ., steatosis).

FLD may arise from a number of sources, including excessive alcohol consumption and metabolic disorders, such as those associated with insulin resistance, obesity, and hypertension. The disease is most prevalent in individuals who are obese or who have diabetes.

In some embodiments of the present invention, alone or in combination with other preferred embodiments, the fatty liver disease, or related condition, is selected from the list consisting of non-alcoholic fatty liver disease (NAFLD), non-alcoholic steatohepatitis (NASH), metabolic associated fatty liver disease (MAFLD), steatosis, steatohepatitis, alcohol related liver disease (ARLD), and any combination thereof. Preferably, the alcohol related liver disease is alcohol fatty liver disease (AFL), alcoholic steatohepatitis (ASH) or alcoholic cirrhosis.

In some embodiments of the present invention, the fatty liver disease is alcohol related liver disease (ARLD) or non-alcoholic fatty liver disease (NAFLD). Preferably, the alcohol related liver disease is alcohol fatty liver disease (AFL), alcoholic steatohepatitis (ASH) or alcoholic cirrhosis.

In some embodiments of the present invention, the fatty liver disease, or related condition, is selected from the list consisting of non-alcoholic fatty liver disease (NAFLD), non-alcoholic steatohepatitis (NASH), metabolic associated fatty liver disease (MAFLD), steatosis steatohepatitis, and any combination thereof. Preferably, AG5, or AG5 in combination with calcifediol, is for use in the treatment and/or prevention of NAFLD and/or NASH.

In alcohol induced fatty liver disease (AFL) initially fat accumulates in liver cells, but then the disease can progress to alcoholic hepatitis which causes the liver to swell and become damaged if the individual continues to consume alcohol. The individual can also develop alcoholic cirrhosis or scarring of the liver which in turn can cause liver failure. Heavy drinkers can progress from AFL to alcoholic hepatitis to alcoholic cirrhosis over time.

Nonalcoholic fatty liver disease (NAFLD) is a fatty liver disorder with histological features of AFL but in individuals who consume little to no alcohol. Like AFL, NAFLD is due to the abnormal retention of fat (lipids) by hepatocytes. Other fatty liver diseases can develop in a patient with other types of liver diseases, such as but not limited to, chronic viral hepatitis C (HCV), chronic viral hepatitis B (HBV), chronic autoimmune hepatitis (AIH), diabetes and Wilson's disease. Fatty liver can also be associated with indications caused by disruptions in lipid metabolism, such as disorders due to drugs, e.g., gastrointestinal disorders (e.g., intestinal bacterial outgrowth, gastroparesis and irritable bowel syndrome), chemotherapy, gastrointestinal surgeries for obesity, malnutrition and genetic defects in proteins that process lipids. A certain population of NAFLD progresses to non-alcoholic steatohepatitis (NASH) and forms liver fibrosis due to hepatic inflammation and hepatocyte death. Thereafter, cirrhosis is induced, which can eventually lead to liver cancer and cardiovascular disease.

NAFLD includes a spectrum of histological forms including hepatic steatosis, and non-alcoholic steatohepatitis (NASH), which is characterized by liver inflammation, steatosis, necrosis and fibrosis due to the disruption of liver cells. NAFLD typically follows a benign, non-progressive clinical course, however, NASH is a potentially serious condition. As many as 25% of NASH patients may progress to advanced fibrosis, cirrhosis and experience complications of portal hypertension, liver failure and hepatocellular carcinoma (Yeh and Brunt, Am J Clin Pathol, 2007, 128(5):837-47).

Nonalcoholic steatohepatitis ("NASH") is a type of fatty liver disease in which fat builds up in the liver of people who drink little or no alcohol. Particularly, NASH is an advanced form of non-alcoholic fatty liver disease (NAFLD). This causes inflammation of the liver and damage to the cells in the liver, which may lead to cirrhosis (scarring of the liver) and liver failure. People with NASH are at increased risk of developing liver cancer. It is more common in middle-aged adults, especially in those who are overweight or obese or who have diabetes or high levels of cholesterol and triglycerides (a type of fat) in the blood.

Metabolic associated fatty liver disease or "MAFLD" (also referred herein as "Metabolic dysfunction-associated steatotic liver disease") is often asymptomatic and usually discovered during routine laboratory tests with altered transaminase levels. This disease comprises two different histological forms: 1) simple steatosis, defined as the presence of fatty droplets, mainly triglycerides, in the liver without hepatocellular necrosis and without or with minimal inflammation; and 2) Metabolic associated steatohepatitis, characterised by fat accumulation, hepatic inflammation and ballooning of hepatocytes with or without fibrosis. Metabolic associated steatohepatitis progression underlies cirrhosis and eventually hepatocellular carcinoma. Accurate diagnosis of metabolic associated steatohepatitis and staging of the level of fibrosis are important for disease management and monitoring of clinical progression to cirrhosis. Metabolic dysfunction-associated steatotic liver disease without fibrosis, or with negligible fibrosis, has a good prognosis with lifestyle modifications, while fibrosis has been proposed as the most important predictive indicator affecting prognosis and the only histopathological parameter closely associated with liver transplantation and liver-related death in patients with metabolic associated steatohepatitis.

As used in the present invention, the term "to treat" or "treatment" comprises: preventing and/or inhibiting the disease or disorder, i.e., stopping its development; relieving the disease or disorder, i.e., causing regression of the disease or disorder; and/or stabilizing the disease or disorder in a subject. In the present invention, the disease or disorder is fatty liver disease or disorder, or a condition caused by fatty liver. The "treatment" of a disease or disorder condition may also lead to a partial response (e.g., amelioration of symptoms) or complete response (e.g., disappearance of symptoms) of the subject/patient suffering from the disease, or disorder. Accordingly, the "treatment" may also refer to an amelioration of the disease or disorder, which may, e.g., lead to a halt in the progression of disease, or disorder, or a delay in the progression of the disease or disorder.

As used herein, the term "prevention" refers to the avoidance of occurrence of the disease or disorder in a subject, particularly when the subject has predisposition for it, but has not yet been diagnosed, or minimize or hinder the occurrence of the disease or disorder in a subject. It may also comprises reducing the risk of developing a disease or disorder in a subject.

In the present invention "subject" means any animal, preferably a mammal, more preferably a primate, in particular a human being, of any breed, sex or age. In a preferred embodiment, alone or in combination with the rest of preferred embodiments, the subject is a human.

### Composition and combined preparation of the invention

AG5 can be comprised in, or it can be administered, as an active compound in the form of a composition. Thus, in other aspect, the invention relates to a composition comprising the AG5 compound, hereinafter the "composition of the invention", for use in the treatment and/or prevention of a fatty liver disease, or disorder, or a condition caused by fatty liver, in a subject, preferably a subject in need thereof.

Furthermore, as previously mentioned, and shown in further detail in the Example section, the inventors have also tested the combination of AG5 with calcifediol, demonstrating that the combination enhances the inhibitory effect of adipogenesis in MSCs.

Calcifediol (also named as "Calcidiol", "25-hydroxyvitamin D3", "25-Hydroxycholecalciferol" or "25OH-D3"; CAS No: 19356-17-3) is a hydroxycalciol that is calciol in which the hydrogen at position 25 has been replaced by a hydroxy group. Calcifediol also relates to the salts, preferably a pharmaceutically acceptable salt, hydrates and solvates thereof.

Thus, a preferred embodiment of the present invention, alone or in combination with other preferred embodiments, refers to the AG5 and calcifediol for use in the treatment and/or prevention of a fatty liver disease or disorder, or a condition caused by fatty liver.

AG5 and calcifediol may be present in a single composition, preferably a in a single pharmaceutical composition, comprising both compounds; or alternatively, said combination may be part of a combined preparation.

Thus, a preferred embodiment, refers to a composition, preferably a pharmaceutical composition, comprising AG5 and calcifediol for use in the treatment and/or prevention of a fatty liver disease or disorder, or a condition caused by fatty liver.

Other aspect refers to a combined preparation ("combined preparation of the invention") comprising AG5 and calcifediol, wherein AG5 and calcifediol are each in a separate formulation, for use in the treatment and/or prevention of a fatty liver disease or disorder, or a condition caused by fatty liver, in a subject, preferably a subject in need thereof. More preferably, the combined preparation comprises a first composition comprising AG5, and a second composition comprising calcifediol.

The term "combined preparation" refers to a preparation in which the two therapeutic agents are not physically together in the same formulation (e.g. composition), so that they may be also available for separate or sequential application.

In a preferred embodiment of the combined preparation of the invention, the AG5 compound and calcifediol are administered simultaneously, separately or sequentially.

The term "fatty liver disease", as well as examples and preferred embodiments have already been described before and applies equally to this aspect as well as all its preferred embodiments.

The composition of the invention, or the combined preparation of the invention, may be formulated for pharmaceutical administration, i.e., forming part of pharmaceutical products to be administered to the subject by any means of administration. Thus, in a preferred embodiment, the composition of the invention, or the combined preparation of the invention is a pharmaceutical composition or preparation ("pharmaceutical composition of the invention").

In a preferred embodiment, the pharmaceutical composition/preparation further comprises at least one pharmaceutically acceptable carrier and/or at least one excipient. In the case of the combined preparation, any of the separate formulations forming the combined preparation can further comprises at least one pharmaceutically acceptable carrier and/or at least one excipient.

The term "excipient" refers to a substance that helps the absorption of any components or compounds of the composition of the invention (or combined preparation of the invention), namely, the AG5 compound and/or calcifediol, or stabilizes the components or compounds and/or assists the preparation of the pharmaceutical composition in the sense of giving it consistency or flavours to make it more pleasant. Thus, the excipients may have the function, by way of example but not limited thereto, of binding the components (for example, starches, sugars or cellulose), sweetening, colouring, protecting the active ingredient (for example, to insulate it from air and/or moisture), filling a pill, capsule or any other presentation or a disintegrating function to facilitate dissolution of the components, without excluding other excipients not listed in this paragraph. Therefore, the term "excipient" is defined as that material that included in the galenic forms, is added to the active ingredients or their associations to enable their preparation and stability, modify their organoleptic properties or determine the physical and chemical properties of the pharmaceutical composition and its bioavailability. The "pharmaceutically acceptable" excipient must allow the activity of components or compounds of the pharmaceutical composition, that is, be compatible with the effect exerted by AG5 compound and/or calcifediol.

The "vehicle" or "carrier" is preferably an inert substance. Carrier functions are to facilitate the incorporation of other components or compounds, allow better dosage and administration and/or give consistency and form to the pharmaceutical composition. Therefore, the carrier is a substance used in the drug to dilute any of the components or compounds of the pharmaceutical composition of the present invention to a given volume or weight; or that even without diluting these components or compounds, it is able to allow better dosage and administration and/or give consistency and form to the drug. When the presentation is liquid, the pharmaceutically acceptable carrier is the diluent. The carrier can be natural or unnatural. Examples of pharmaceutically acceptable carriers include, without being limited thereto, water, salt solutions, alcohol, vegetable oils, polyethylene glycols, gelatine, lactose, starch, amylose, magnesium stearate, talc, surfactants, silicic acid, viscous paraffin, perfume oil, monoglycerides and diglycerides of fatty acids, fatty acid esters petroetrals, hydroxymethylcellulose, polyvinylpyrrolidone and the like.

Furthermore, the excipient and the carrier must be pharmacologically acceptable, i.e., the excipient and the carrier are permitted and evaluated so as not to cause damage to the subject to whom it is administered.

The various pharmaceutical forms of administration of pharmaceutical compositions and the excipients necessary to obtain them are well known to the expert in the field.

When in the present invention AG5 is combined with calcifediol, each component of the pharmaceutical composition (i.e. AG5 and calcifediol) can be administered simultaneously, separately or sequentially, in a therapeutically effective amount of the combination of the two compounds. For example, when each component AG5 and calcifediol are in a single composition, they are administered at the same time. In the case of the combined preparation, the therapeutic agents can be administered separately, sequentially or simultaneously.

As understood by the person skilled in the art, the AG5 compound, have to be present in an effective amount, preferably in a therapeutically effective amount, in order to exert their effects, such as the effects disclosed in the Example section. Likewise, when calcifediol is used in combination with AG5 compound (either in the composition of the invention, preferably the pharmaceutical composition of the invention, or in the combined preparation of the invention), AG5 and calcifediol are present in an effective amount, preferably in a therapeutically effective amount. An effective amount can be the amount of the compound/s which produces a reduction or decrease of fatty vesicle accumulation in hepatic cells. An assay to assess whether the amount of the compound/s is effective amount for exerting their effects can be found in the Examples of the present description, particularly in Example 1, section 2.2.

In a preferred embodiment, the composition comprising AG5 and calcifediol, or the combined preparation of the invention, comprises a therapeutically effective amount of the combination of AG5 and calcifediol.

The effective amount may vary depending on, for example, the age, body weight, general health, sex and diet of the subject, as well as on the mode and time of administration, the excretion rate or any potential co-treatment with other drugs.

The compositions/formulations of the present invention can be formulated for administration to an animal, and more preferably to a mammal, including a human, in a variety of forms known in the prior art. Thus, they may be, without limitation, in aqueous or non-aqueous solutions, in emulsions or in suspensions. Examples of non-aqueous solutions are, for example, but without limitation, propylene glycol, polyethylene glycol, vegetable oils, such as olive oil, or injectable organic esters, such as ethyl oleate. Examples of aqueous solutions are, for example, but not limited to, water, alcoholic solutions in water, or saline media. Aqueous solutions may be buffered or unbuffered and may have additional active or inactive components. Additional components include salts to modulate ionic strength, preservatives including, but not limited to, antimicrobial agents, antioxidants, chelators, or the like, or nutrients, including glucose, dextrose, vitamins, and minerals. Alternatively, the compositions/formulations can be prepared for administration in solid form (for example, without limitation to, powder or granular form). The compositions/formulations may be combined with various vehicles or inert excipients, including but not limited to binders, such as microcrystalline cellulose, gum tragacanth, or gelatin; excipients, such as starch or lactose; dispersing agents, such as alginic acid or corn starch; lubricants, such as magnesium stearate, glidants such as colloidal silicon dioxide; sweetening agents, such as sucrose or saccharin; or flavouring agents, such as peppermint or methyl salicylate.

Additionally, the composition of the invention, or the formulations forming the combined preparation of the invention, may comprise an adjuvant. "Adjuvant" means any substance which enhances the effectiveness of the pharmaceutical composition/preparation of the invention. Examples of adjuvants include, but are not limited to, metabolites of the endocrine system of vitamin D (such as cholecalciferol, ergocalciferol, calcifediol and calcitriol or their analogues), omega-3 fatty acids (such as alpha-linolenic acid (ALA), eicosapentaenoic acid (EPA) and docosahexaenoic acid (DHA)), adjuvants consisting of aluminium (alum) salts, such as aluminium hydroxide, aluminium phosphate or aluminium sulphate, oil-in-water or water-in-oil emulsion formulations such as Complete Freund's Adjuvant (ACF) as well as Incomplete Freund's Adjuvant (AIF), mineral gels, gels, block copolymers, Avridine^{™}, SEAM62, adjuvants consisting of bacterial cell wall components such as adjuvants including liposaccharides (e.g. lipid A or Monophosphoryl Lipid A (MLA), trehalose dimycolate (TDM), and cell wall skeleton components (CWS), *heat shock* proteins or their derivatives, adjuvants derived from ADPribosylated bacterial toxins, including diphtheria toxin (DT), pertussis toxin (PT), cholera toxin (CT), the heat labile toxins of *E.coli heat* labile toxins (LT1 and LT2), Endotoxin A and *Pseudomonas* exotoxin, B. *cereus* exoenzyme B, *B*. *sphaerieus* toxin, *C*. *botulinum* toxins C2 and C3, *C*. *limosum* exoenzyme as well as the toxins of *C*. *perfringens, C. spiriforma* and *C*. *diffieile, S. aureus,* EDIM and toxin mutants such as CRM-197, non-toxic mutant diphtheria toxin; saponins such as ISCOMs (immunostimulatory complexes), chemokines and cytokines such as interleukins (IL-I IL-2, IL-4, IL-5, IL-6, IL-7, IL-8, IL-12, etc), interferons (such as interferon gamma), macrophage colony-stimulating factor (M-CSF), tumour necrosis factor (TNF), defensins 102, RANTES, MIPI-alpha, and MEP-2, muramyl peptides such as N-acetyl-muramyl-L-threonyl-D-isoglutamine (thr-MDP), N-acetyl normuramyl-L-alanyl-D-isoglutamine (nor-MDP), N-acetylmuramyl-L- alanyl-Disoglutaminyl- L-alanine-2-( 1 '-2'-dipalmitoyl-s- n-glycero-3 huydroxyphosphoryloxy)ethylamine (MTP-PE) etc; adjuvants derived from the CpG family of molecules, synthetic CpG dinucleotides and oligonucleotides comprising CpG motifs, lysosum exoenzyme from *C*. *Limosum* and synthetic adjuvants such as PCPP, cholera toxin, Salmonella toxin, alum and similar, aluminium hydroxide, N-acetyl-muramyl-L-threonyl-D-isoglutamine (thr-MDP), N-acetyl-nor-muramyl-L-alanyl-D-isoglutamine, MTP-PE and RIBI, which contains three components extracted from bacteria, monophosphoryl lipid A, trehalose dimycolate and cell wall skeleton (MPL+TDM+CWS) in a 12%/Tween 80 squalene emulsion.

The compositions/preparations of the invention are manufactured by methods known in the art

In another preferred embodiment, alone or in combination with other preferred embodiments, the AG5 compound, the composition of the invention, or the combined preparation of the invention, is administered parenterally (including subcutaneously, intraperitoneally, intradermally, intramuscularly, intravenously, etc.), orally, topically, rectally, or by inhalation. The various pharmaceutical forms of administration of medicinal products and the excipients necessary to obtain them are well known to the person skilled in the art.

The term "administration", as used herein, refers to the introduction of the therapeutic agent/s or active ingredient/s (i.e. AG5 and/or calcifediol), alone or in a composition/formulation, preferably a pharmaceutical composition/formulation, into a subject.

Furthermore, as understood by a person skilled in the art, a healthcare professional can adjust the concentration of the active ingredient/s for use in the treatment and/or prevention of a fatty liver disease or disorder (or a condition caused by fatty liver), but not limited to, following therapeutic and toxicity criteria.

In a preferred embodiment, alone or in combination with other preferred embodiments, the AG5 compound is administered at a concentration, or dose, ranging from 0.0001 mg/(kg h) to 10 mg/(kg h) (including both ends values).

In another preferred embodiment, alone or in combination with other preferred embodiments, the calcifediol is administered at a concentration, or dose, ranging from 0.001 mg/(kg h) to 0.3 mg/(kg h) (including both ends values).

In a more preferred embodiment, the AG5 compound is administered at 0.005 to 1 mg/(kg h) and calcifediol is administered at 0.01 to 0.06 mg/(kg h).

In another preferred embodiment, alone or in combination with other preferred embodiments, AG5:calcifediol moles ratio in the composition of the invention or combined preparation of the invention, preferably the pharmaceutical composition, is 500:3 to 50000:3.

In another preferred embodiment, alone or in combination with other preferred embodiments, the AG5 compound is administered parenterally or orally.

In another preferred embodiment, alone or in combination with other preferred embodiments, the calcifediol is administered parenterally or orally.

### Use of the AG5 compound in the manufacture of a medicament

In other aspect, the invention relates to the use of AG5 compound, the composition of the invention, or the combined preparation of the invention, in the manufacture of a medicament, or in the manufacture of a pharmaceutical composition, for the treatment and/or prevention of a fatty liver disease or disorder, or a condition caused by fatty liver, in a subject, preferably a subject in need thereof. Techniques and procedures for the production of medicaments are widely known in the state of the art.

The terms used to define the present aspect of the invention have been previously explained, and they and their preferred embodiments are applicable to the present aspect of the invention.

### Method of treatment of the invention

In another aspect, the invention relates to a method for the treatment and/or prevention of a fatty liver disease or disorder, or a condition caused by fatty liver, comprising the administration of AG5 compound, the composition of the invention, or the combined preparation of the invention to a subject, preferably a subject in need thereof.

In a preferred embodiment, the method further comprises a step, previous to the AG5 administration, of identifying a subject suffering from a fatty liver disease or disorder, or a condition caused by fatty liver.

The terms used to define the present aspect of the invention have been previously explained, and they and their preferred embodiments are applicable to the present aspect of the invention.

### DESCRIPTION OF THE DRAWINGS

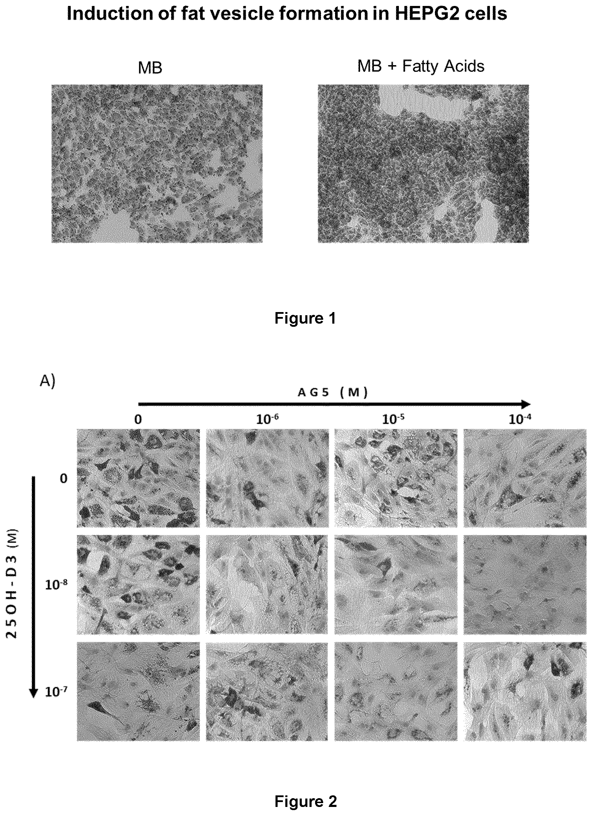

**Figure 1****.** HEPG2 cell cultures were maintained for 6 hours in DMEM medium supplemented with 1% BSA (MB) and then maintained for 24 hours in the same medium or in a medium supplemented with a concentration of 600 µM of a mixture of oleic acid and palmitic acid (2:1) (MB + Fatty Acids). The images correspond to optical microscope photographs obtained at 200x of cultures maintained in MB or MB + Fatty Acids and subsequently stained with Oil Red O and hematoxylin to show the presence of fat vesicles. In the image of cultures treated with the fatty acid mixture, a significant increase in staining is observed due to the greater accumulation and formation of fat vesicles.

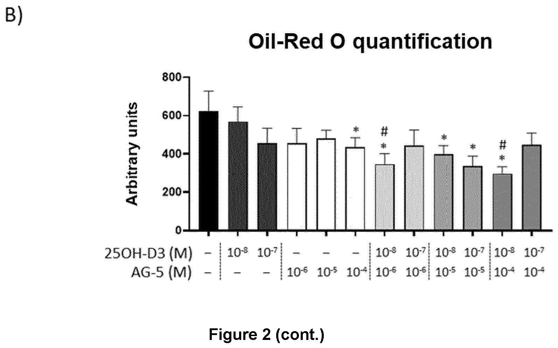

**Figure 2****.** Oil Red O staining for fat vesicles. Cultures were stained at 14 days after the start of adipogenic differentiation in the presence of different combinations of calcifediol (25OH-D3) 10⁻⁸ and 10⁻⁷ M and/or AG5 at 10⁻⁶, 10⁻⁵, and 10⁻⁴ M. A) Representative images under the microscope (200x) of MSCs induced into adipocytes in the presence of various treatments for 14 days and stained with Oil Red O. B) Graphical representation of the quantification of Oil Red O staining for the different treatments. *p<0.05 vs Control; #p<0.05 vs the same concentration of 25OH-D3.
**Figure 3****.** Gene expression of adipogenesis markers PPARG2, LPL, ATGL, FASN, and FABP4 at 7 days of adipogenic differentiation in MSCs in the presence or absence of different combinations of calcifediol (25OH-D3) 10⁻⁸ and 10⁻⁷ M and/or AG5 at 10⁻⁶, 10⁻⁵, and 10⁻⁴ M. *p<0.05 vs Control; #p <0.05 vs the same concentration of 25OH-D3; +p <0.05 vs the same concentration of AG-5.

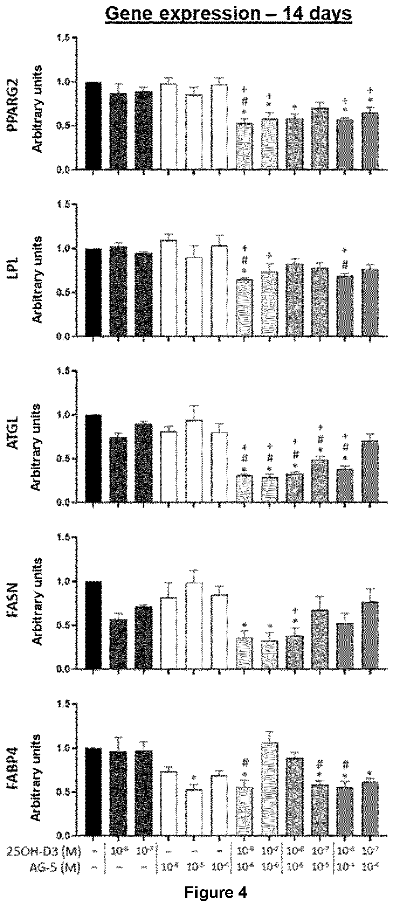

**Figure 4****.** Gene expression of adipogenesis markers PPARG2, LPL, ATGL, FASN, and FABP4 at 14 days of adipogenic differentiation in the presence or absence of different combinations of calcifediol (25OH-D3) 10⁻⁸ and 10⁻⁷ M and/or AG5 at 10⁻⁶, 10⁻⁵, and 10⁻⁴ M. *p<0.05 vs Control; #p<0.05 vs the same concentration of 25OH-D3; +p<0.05 vs the same concentration of AG-5.
**Figure 5****.** Oil Red O staining for fat vesicles in HEPG2 cultures. Cultures were stained 24 hours after the start of fat vesicle formation induction in a medium supplemented with fatty acids, in the presence of different combinations of calcifediol (25OH-D3) 10⁻⁸ and 10⁻⁷ M and/or AG5 at 10⁻⁶, 10⁻⁵, and 10⁻⁴ M. A) Representative images under the microscope (200x) of HEPG2 cultures subjected to various treatments and subsequently stained with Oil Red O. B) Graphical representation of the quantification of Oil Red O staining for the different treatments. *p<0.05 vs Control.
**Figure 6****.** Gene expression of adipogenic markers PPARG2, ATGL, and FASN in HEPG2 cell cultures induced to form fat vesicles in medium supplemented with oleic acid and palmitic acid, in the presence or absence of different combinations of calcifediol (25OH-D3) 10⁻⁸ and 10⁻⁷ M and/or AG5 at 10⁻⁶, 10⁻⁵, and 10⁻⁴ M. *p<0.05 vs Control.

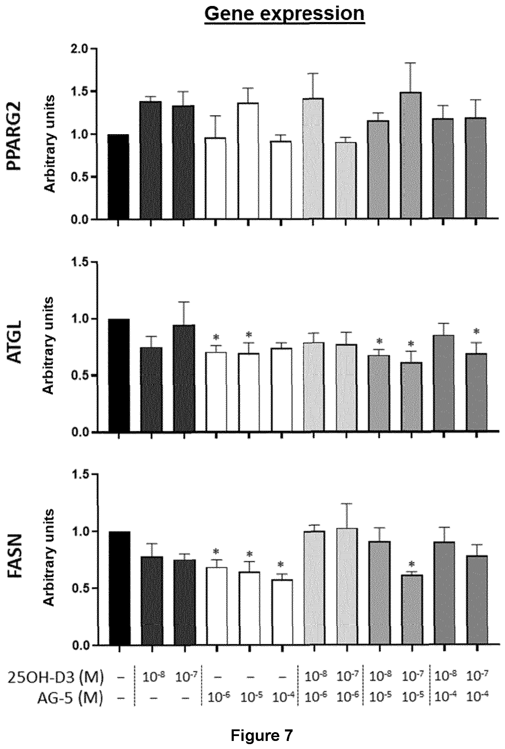

**Figure 7**. Gene expression of adipogenic markers PPARG2, ATGL, and FASN in HEPG2 cell cultures induced to form fat vesicles in medium supplemented with oleic acid and palmitic acid for 24 hours and then treated for another 24 hours with the different combinations of calcifediol (25OH-D3) 10⁻⁸ and 10⁻⁷ M and/or AG5 at 10⁻⁶, 10⁻⁵, and 10⁻⁴ M. *p<0.05 vs Control.

### Examples

In the following, the invention will be illustrated by means of tests carried out by the inventors, which demonstrate the effectiveness of the invention.

### Example 1

The effect of the compound AG5, with or without calcifediol, on the formation of fat vesicles and the regulation of adipogenic genes was studied in two types of cell lines. The study consists of the following parts:
1- Evaluate the effect of 14-deoxy-12(R,S)-sulfo-andrographolide (AG5) and/or calcifediol on adipogenic differentiation in human mesenchymal stromal cells (MSCs) derived from bone marrow.
2- Study the effect of AG5 and/or calcifediol on a steatosis model in HEPG2 cells.

### 1. Materials and methods

### 1.1. Cell cultures and adipogenic differentiation.

The human bone marrow-derived mesenchymal stromal cells (MSCs) used were from previously characterized cryopreserved cells from the GC-17 research group's cell line collection. The MSCs were thawed and cultured in alpha-Minimum Essential Medium (α-MEM, Biowest Nuaillé, France) containing 2 mM UltraGlutamine (Biowest), 10% fetal bovine serum (FBS) (Corning, Glendale, AZ, USA), 100 U of ampicillin, 0.1 mg of streptomycin/mL, and 1 ng of basic fibroblast growth factor (bFGF)/mL (Sigma-Aldrich, Saint Louis, MO, USA). Cells were maintained at 37°C in a 5% CO₂ atmosphere with 95% humidity. The culture medium was changed every 3 or 4 days. Once the culture reached near 90% confluence, the cells were harvested using trypsin-EDTA (Biowest) and seeded in culture plates (Nalgene-Nunc-Thermo Fisher Scientific) at density of 3,000 cells/cm². When the culture reached between 70-90% confluence, cells were induced to differentiate into adipocytes with the following adipogenic medium: culture medium without bFGF supplemented with 5×10⁻⁷ M dexamethasone, 50 µM indomethacin, and 0.5 mM isobutylmethylxanthine.

The human hepatocyte cell line HEPG2 was cultured in Dulbecco's Modified Eagle Medium (DMEM, Biowest Nuaillé, France), supplemented with 2 mM UltraGlutamine, 10% FBS, 100 U of ampicillin, and 0.1 mg of streptomycin/mL under the same culture conditions as described for MSCs. These cells tend to accumulate small fat vesicles under basal culture conditions. However, this process can be intensively induced by supplementing the medium with fatty acids, leading to a significant increase in the accumulation of cytoplasmic fat vesicles. When the cells reached 80-90% confluence, the medium was removed and replaced with DMEM medium without FBS, supplemented with 1% bovine serum albumin (BSA, Panreac-Applichem, Barcelona, Spain). Cells were maintained in this medium for 6-18 hours, after which the medium was replaced with the same medium supplemented with 600 µM of a mixture of oleic and palmitic acid (2:1). The cells were exposed to this medium for 24 hours to stimulate fat vesicle formation. After this time, the cells were lysed for RNA isolation for gene expression studies or fixed and stained with Oil Red O to reveal fat vesicle accumulation. As a control, cells were maintained in DMEM medium + 1% BSA without fatty acids (basal medium) (Figure 1).

### Treatments

MSCs and HEPG2 cells were subjected to the following treatments:
1- Calcifediol 10⁻⁸ M
2- Calcifediol 10⁻⁷ M
3- AG5 10⁻⁶ M
4- AG5 10⁻⁵ M
5- AG5 10⁻⁴ M
6- AG5 10⁻⁶ M + Calcifediol 10⁻⁸ M
7- AG5 10⁻⁶ M + Calcifediol 10⁻⁷ M
8- AG5 10⁻⁵ M + Calcifediol 10⁻⁸ M
9- AG5 10⁻⁵ M + Calcifediol 10⁻⁷ M
10- AG5 10⁻⁴ M + Calcifediol 10⁻⁸ M
11- AG5 10⁻⁴ M + Calcifediol 10⁻⁷ M

In the case of MSCs, treatments were added at the beginning of adipogenic induction and maintained for the 14 days of differentiation. HEPG2 cells were subjected to two treatment patterns: co-treatment and post-treatment. In the first pattern, treatments were added at the moment of treating the cells with fatty acids to induce fat vesicle accumulation and maintained for 24 hours of induction. The post-treatment consisted of inducing fat formation for 24 hours and then treating for an additional 24 hours with various combinations of AG5 and calcifediol.

AG5 was provided by the promoter in powder form. For application to the cultures, a 0.1 M stock solution was prepared in phosphate-buffered saline (PBS). Calcifediol was obtained from Sigma-Aldrich (reference H-083-1ML) as a solution in ethanol at 100 µg/mL (2.5×10⁻⁴ M). Stock solutions of both products were diluted to working concentrations with the respective culture medium for each treatment.

### 1.2. Oil Red-O staining

The formation of fat vesicles in cultures induced to differentiate into adipocytes was evaluated using Oil Red O staining. The cultures were fixed with 3.7% formaldehyde for 15 minutes. Then, they were stained with a solution consisting of 40% distilled water and 60% Oil Red-O (0.35% (w/v) in isopropanol). After 20 minutes of incubation, the cells were washed with distilled water and stained with hematoxylin. To quantify staining, at least nine images were randomly taken from each well of P24 culture plates using an optical microscope at 200x. The images were analyzed using imaged software (version 1.53f51; NIH, Bethesda, MD, USA; https://imagej.nih.gov/ij) to calculate the area occupied by the staining. The value obtained was normalized to the number of cells in each image (Oil Red O area/number of cells).

### 1.3. Quantification of Adipogenic Gene Expression

RNA from cultures was obtained using the NZY Total RNA Isolation kit from NZYTech (Lisbon, Portugal), following the manufacturer's instructions. RNA was quantified with a NanoDrop ND-1000 spectrophotometer (Thermo Fisher Scientific), and 900 ng were reverse-transcribed into cDNA using an iScript cDNA synthesis kit from Bio-Rad (Hercules, Spain). Quantitative real-time PCR (QRT-PCR) was performed on a Biorad CFX96 Connect Real-Time PCR Detection System. Each PCR reaction was performed in a volume of 10 µL containing 1 µL of cDNA, 10 pmoles of each primer pair (Table 1), and 1X NZYSpeedy qPCR Green Master Mix (NZY). The PCR amplification program consisted of one cycle at 95°C for 2 minutes (activation of DNA polymerase) followed by 40 to 45 cycles of 95°C for 5 seconds (DNA denaturation) and 65°C for 30 seconds (primer annealing and DNA polymerase extension). Results were analyzed with Bio-Rad CFX Maestro software. The expression of the gene encoding RNA polymerase II subunit A (POLR2A) was used as a housekeeping gene to normalize and calculate the relative expression of the evaluated genes.

**Table 1. Primer sequences and PCR product sizes**

| ***Gen*** | ***Primer sequence (5'→ 3')*** | ***Amplicon (bp)*** |
|---|---|---|
| Peroxisome Proliferator-activated receptor gamma 2 (***PPARG2***) | GCGATTCCTTCACTGATACACTG (SEQ ID NO: 1) | 136 |
| | GAGTGGGAGTGGTCTTCCATTAC (SEQ ID NO: 2) | |
| Lipoprotein lipase (***LPL***) | AAGAAGCAGCAAAATGTACCTGAAG (SEQ ID NO: 3) | 113 |
| | CCTGATTGGTATGGGTTTCACTC (SEQ ID NO: 4) | |
| Fatty-acid-binding protein **4** (***FABP4***) | TCAGTGTGAATGGGGATGTGAT (SEQ ID NO: 5) | 162 |
| | TCTGCACATGTACCAGGACACC (SEQ ID NO: 6) | |
| Adipose triglyceride lipase (***ATGL***) | CCAACACCAGCATCCAGTTCA (SEQ ID NO: 7) | 102 |
| | ATCCCTGCTTGCACATCTCTC (SEQ ID NO: 8) | |
| Fatty acid synthase (***FASN***) | AAGCTGAAGGACCTGTCTAGG (SEQ ID NO: 9) | 146 |
| | CGGAGTGAATCTGGGTTGATG (SEQ ID NO: 10) | |
| Polymerase (RNA; DNA directed) II polypeptide A (***POLR2A***) | TTTTGGTGACGACTTGAACTGC (SEQ ID NO: 11) | 125 |
| | CCATCTTGTCCACCACCTCTTC (SEQ ID NO: 12) | |

### 2. Results

### 2.1. Effect of AG5 and calcifediol on MSC adipogenic differentiation

The effect on the accumulation of fat vesicles in MSCs differentiated into adipocytes in the presence of different combinations of concentrations of 25OH-D3 and AG5 during 14 days is shown in Figure 2. All treatments tended to reduce fat accumulation. In the case of the highest concentration of AG5 (10⁻⁴ M), the reduction was significant, as well as in certain combinations of AG5 and calcifediol. Specifically, for the combinations AG5 10⁻⁶ M + 25OH-D3 10⁻⁸ M, AG5 10⁻⁵ M + 25OH-D3 10⁻⁸ M, AG5 10⁻⁵ M + 25OH-D3 10⁻⁷ M, and AG5 10⁻⁴ M + 25OH-D3 10⁻⁸ M. Although the first and last combinations mentioned had a greater effect than when treated with the corresponding concentration of calcifediol alone. However, in none of them was the reduction in fat vesicle formation greater than that produced by treatments with the evaluated concentrations of AG5 alone.

The gene expression of adipogenic markers PPARG2, LPL, ATGL, FASN, and FABP4 (serve as markers for adipogenic differentiation) was evaluated at 7 and 14 days after the induction of differentiation and treatments. At 7 days of differentiation, in a stage considered as preadipocytes, no significant changes in expression were observed between the different treatments for the PPARG2, LPL, and FABP4 genes.

However, in the expression of the genes ATGL and FASN, a reduction in mRNA levels was observed relative to untreated cells with several treatments, especially in those combining AG5 at concentrations of 10⁻⁶ and 10⁻⁵ M plus calcifediol (Figure 3). Of these two genes, ATGL showed the most significant inhibition. The inhibition was approximately 50% with treatments of 10⁻⁶ and 10⁻⁵ M of AG5 and 10⁻⁸ M of calcifediol, and around 80% with the previously described combinations of AG5 and calcifediol (Figure 3).

At 14 days of differentiation, a stage where mature adipocytes can be observed in the cultures, an inhibition of expression was seen in all the studied genes, mainly in treatments involving AG5 plus calcifediol, particularly the combination of AG5 10⁻⁶ M with 10⁻⁸ or 10⁻⁷ M of calcifediol (Figure 4). Treatment with calcifediol alone or AG5 at the evaluated concentrations did not produce significant changes in mRNA levels of any of the studied genes, except for FABP4, whose expression significantly decreased with AG5 10⁻⁵ M treatment (Figure 4).

### 2.2. Effect of AG5 and calcifediol on fat vesicle accumulation in HEPG2 cells

The presence of AG5 and/or calcifediol in the culture medium during the induction of fat vesicle formation in HEPG2 cells resulted in a decrease in the accumulation of these vesicles with all evaluated treatments, with significant differences when AG5 was present in any of the evaluated treatments (Figure 5). However, the combined treatment of AG5 and calcifediol at any of the studied concentrations did not produce a greater effect compared to treatments with only AG5, according to the results obtained from the quantification of Oil Red O staining in these cultures (Figure 5B).

Gene expression studies of the genes PPARG2, ATGL, and FASN in these cultures show that the treatments did not produce significant changes in the first two genes.

No significant change was observed in the expression of the gene encoding the transcription factor that induces adipogenesis (PPARG2). For the gene encoding ATGL, significant decreases in expression were only observed in treatments where AG5 was present at the lowest evaluated concentration (10⁻⁶ M) (Figure 6). However, all treatments resulted in inhibition of the expression of the gene encoding fatty acid synthase (FASN), without significant differences between them (Figure 6).

The effect of the different treatments on the accumulation of fat vesicles in HEPG2 cultures was also reflected in the expression of fat metabolism-related genes ATGL and FASN (Figure 7). A decrease in expression was observed with the various treatments, being more significant with AG5 treatments at different concentrations in the absence of calcifediol. The presence of calcifediol did not produce a greater effect of AG5 on HEPG2 under these conditions (Figure 7). The expression of the PPARG2 gene, as with co-treatment conditions, was not significantly affected by any of the evaluated treatments (Figure 7).

### 3. Conclusions

Based on the results, the following conclusions can be drawn:
1- Calcifediol and AG5, when applied individually to MSCs induced to differentiate into adipocytes, reduce fat vesicle formation, specially at the highest evaluated concentrations (10⁻⁷ M and 10⁻⁴ M, respectively).
2-The combined application of calcifediol and AG5 enhances the inhibitory effect of these compounds on adipogenesis in MSCs.
3-Gene expression studies in MSCs correlate with the observed phenotype. The combined treatments of calcifediol and AG5 result in a more significant inhibition of adipogenic gene expression than individual treatments.
4-The expression of the primary transcription factor that induces adipogenesis, PPARG2, is not significantly influenced by the different treatments at 7 days of adipogenic differentiation in MSCs, while genes related to fat metabolism, such as those encoding adipose triglyceride lipase (ATGL) and fatty acid synthase (FASN), are primarily inhibited by the combined treatments of calcifediol and AG5. This suggests that the treatments' effect likely acts on the fat metabolism of preadipocytes rather than on the adipogenic differentiation signaling pathways.
5- After 14 days of adipogenic differentiation in MSCs, the combined effect of calcifediol and AG5 negatively affected the expression of all five studied adipogenic genes. This indicates that the inhibitory effect on adipogenesis of these treatments occurs mainly at the late stages of adipogenesis.
6-The treatment of HEPG2 cells with various combinations of AG5 and/or calcifediol reduces fat vesicle accumulation. Particularly, a significant decrease in the accumulation of fat vesicles during the induction of fatty vesicle formation in HEPG2 cells, with significant differences was demonstrated when AG5 was present.
7-The inhibition of the expression of genes involved in fat metabolism, particularly the gene encoding fatty acid synthase (FASN), in HEPG2 cells treated with the various combinations of AG5 and calcifediol indicates that these compounds interfere with metabolic processes directed toward fat vesicle accumulation.

### Example 2. AG5 synthesis/obtention

AG5 has been synthesized, purified and characterized starting from andrographolide. Briefly, andrographolide (10 g) was dissolved in 200 mL of 95% ethanol on heating at reflux (1 hour, to ensure complete dissolution of the andrographolide) (solution 1). To 40 mL of Na₂SO₃ 1M, 48 mL of 2% H₂SO₄ (wt/wt) and 80 mL of water was added (solution 2). Solution 1 (at 75 °C) was poured into solution 2 (at 75°C) and refluxed for one hour. The reaction solution was allowed to cool up to room temperature. The pH value of the reaction solution was adjusted to 6-7 by adding 2% H₂SO₄ (v/v), and the solution was led to dryness in a rotary evaporator under reduced pressure (67 mbar) and water bath (60 °C). Subsequently, the residue was dissolved with 100 mL water, followed by a three-fold extraction with same chloroform volume (3 × 100 mL). Then, the isolated aqueous layer was evaporated to dryness in a rotary evaporator (67 mbar, 60° C). This residue was re-dissolved in 100 mL of methanol and filtered using a Buchner Funnel Vacuum Filtration with a Whatman filter paper 42. The supernatant was evaporated to dryness in a rotary evaporator (337 mbar, 60° C), freeze-dried (-55 °C, 16 h) and finally purified by High Performance Flash Chromatography (HPFC) in a Biotage^{®} Selekt unit, by using a reversed-phase column (Biotage^{®} Sfär C18 D Duo 100 Å 30 µm 120 g). Separation of the pure compound was carried out with acetonitrile 100% (250 mL/min, 45 min). The recovered liquid phase was evaporated under reduced pressure (223 mbar, RT) and finally freeze-dried (-55 °C, 16 h), yielding a white powder (14-deoxy-12(R,S)-sulfo andrographolide, yield = 30-35%).

¹H-RMN (300 MHz, DMSO-d₆) *δ*: 7.49 (s, 1H), 5.01 (sₐ, 1H), 4.90-4.73 (m, 4H), 4.10 (sₐ, 1H), 3.82 (d, *J =* 10.8 Hz, 1H), 3.45 (d, *J =* 11.7 Hz, 1H), 3.19 (m, 2H), 2.30 (m, 1H), 2.13 (t, *J* = 12.8 Hz, 1H), 1.85-1.52 (m, 6H), 1.25 (m, 2H), 1.04 (s, 3H), 1.01 - 0.83 (m, 2H), 0.60 (s, 3H).

¹³C-RMN (75 MHz, DMSO₆) *δ*: 174.05, 147.38, 147.24, 131.51, 107.42, 78.31, 70.35, 62.61, 54.39, 53.34, 52.67, 42.23, 38.39, 37.88, 36.37, 27.84, 26.96, 23.90, 22.93, 14.79.

QTof-MS (ESI, m/z) of: [M+H]⁺ calcd for C₂₀H₃₁O₇S, 415.1791; found 415.1794.

AG5 compound obtention is also disclosed in ES2908500A1.

## Claims

1. AG5 compound 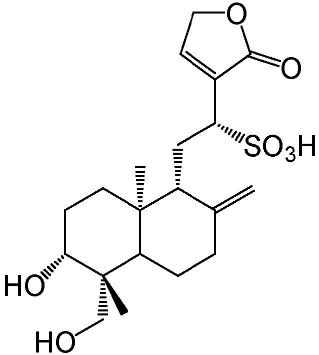 for use in the treatment and/or prevention of a fatty liver disease or disorder in a subject.

2. Composition comprising AG5 compound, for use in the treatment and/or prevention of a fatty liver disease or disorder in a subject.

3. Composition for use according to claim 2, wherein the composition further comprises calcifediol.

4. Composition for use according to claim 2 or 3, wherein the composition is a pharmaceutical composition.

5. Combined preparation comprising AG5 and calcifediol, wherein AG5 and calcifediol are each in a separate formulation, for use in the treatment and/or prevention of a fatty liver disease or disorder in a subject.

6. Combined preparation for use according to claim 5, wherein AG5 compound and calcifediol are administered simultaneously, separately or sequentially.

7. Composition for use according to claim 4, or combined preparation for use according to claim 5 or 6, wherein said pharmaceutical composition or the separate formulations forming the combined preparation, further comprises at least one pharmaceutically acceptable carrier and/or at least one excipient.

8. AG5 compound for use according to claim 1, composition for use according to any one of claims 2, 3, 4, or 7, or combined preparation for use according to any one of claims 5 to 7, wherein the fatty liver disease or disorder is selected from the list consisting of non-alcoholic fatty liver disease (NAFLD), non-alcoholic steatohepatitis (NASH), metabolic associated fatty liver disease (MAFLD), steatosis, steatohepatitis, alcohol related liver disease (ARLD), and any combination thereof.

9. AG5 compound for use according to claim 1 or 8, composition for use according to any one of claims 2, 3, 4, 7 or 8, or combined preparation for use according to any one of claims 5 to 8, wherein the AG5 compound is administered at a concentration ranging from 0.0001 to 10 mg/(kg h), both ends included.

10. AG5 compound for use according to any one of claims 1, 8 or 9, composition for use according to any one of claims 2, 3, 4, 7, 8 or 9, or combined preparation for use according to any one of claims 5 to 9, wherein the AG5 compound is administered parenterally or orally.

11. Composition for use according to any one of claims 3, 4, 7, 8, 9 or 10, or combined preparation for use according to any one of claims 5 to 10, wherein the calcifediol is administered at a concentration ranging from 0.001 to 0.3 mg/(kg h), both ends included.

12. Composition for use according to any one of claims 3, 4, 7, 8, 9, 10 or 11, or combined preparation for use according to any one of claims 5 to 11, wherein the calcifediol is administered parenterally or orally.
